(19) Europäisches Patentamt
European Patent Office
Office européen des brevets

(11) **EP 2 669 711 A2**

## (12) EUROPEAN PATENT APPLICATION

(43) Date of publication:
**04.12.2013 Bulletin 2013/49**

(51) Int Cl.:
***G01T 1/24*** *(2006.01)*

(21) Application number: **13169010.9**

(22) Date of filing: **23.05.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **31.05.2012 EA 201200797**

(71) Applicant: **NIPK "Electron", Co
197758 pos. Pesochny, Saint-Petersburg (RU)**

(72) Inventor: **Kosarev, Ruslan Nikolaevich
188485 Kingisepp, Leningrad region (RU)**

(74) Representative: **Spengler, Robert
Küfergasse 11
89073 Ulm (DE)**

(54) **Method for determination of geometrical sensor shifts in flat panel x-ray image detectors**

(57) The invention relates to the method for measuring of geometrical shift in flat panel x-ray image sensors using a test device.

A test device comprising at least two edge test devices is placed on the detector's operating surface, The test device is exposed to x-rays in order to get its x-ray image where ROIs having pixels coordinates corresponding to the edge of each test device are identified. The pixel coordinates are used to determine sensor geometrical shifts considering minimum value of an objective function.

Technical result involves expansion of technical means of definite application and a possibility to measure sensor geometrical shift with sufficient accuracy

Fig. 1

**Description**

*Field of the invention*

The invention relates to the area of digital X-ray image processing in particular, to the method for measuring of geometrical shift in flat panel x-ray image sensors using a test device.

*Previous state of art*

At present different manufacturers of medical equipment are developing flat panel x-ray image detectors with a field of view up to a few tens of centimeters in size. Some of such detectors contain several sensors inflexibly fixed on a general substrate. For example, in the patent [US № 6895077, issued 17.05.2005] is described an x-ray apparatus comprising a detector consisting of four (2x2) or nine (3x3) CCDs as a possible version. In the [patent US № 7663115, issued 16.02.2010] is described a detector consisting of six CMOS having 20x30 cm field of view. In the x-ray image obtained by such a compound detector in the area of butt-joint between the elements of such a detector there is a possibility of various artifacts which can be caused by the following reasons: 1). Sensors differ from each other in their light-sensitive features; 2). In an ideal detector there must not be any gaps in between sensors and each sensor's column (row) must be aligned with the corresponding column (row) of the neighbouring sensors. It is clear that in real detectors sensors will always have a geometrical shift against its ideal position. This fact negatively affects image quality as well.

These factors cause remarkable artifacts in images that need correcting. In order to arrange accurate correction it is important to understand the nature of these artifacts and to carry out some additional measurements characterizing said artifacts.

Among different image correction techniques, for example, a method for correction of butting artifacts in x-ray images [patent US № 8073191, issued 06.12.2011] based on using a multiple hypothesis hidden Markov model. In the description of the claimed technical solution one shows that the width of the artifact region may achieve a few pixels but general attention is paid to correction of the artifacts as such.

A flat panel detector is an all-of-a-piece device that does not allow direct measuring shifts in between sensors. So, two methods for measurement of geometrical shifts are possible. The first method comprising direct measuring of sensor shifts uses measurement equipment at the stage of detector assembling. For example, optical microscope Galileo AV350 [Galileo AV350 Multi-Sensor Vision System, the L.S. Starrett Company] allows measuring of distances to few microns. The second method involves measurement of sensor shifts in an x-ray image of a test device.

A disadvantage of direct measurement of sensor shifts consists in: 1) difference between sensor positions in the assembled detector and those in the knocked-down one due to mechanical stress; 2) that if there is a necessity to put the assembled detector to measurements, it is to be dismantled in a special room. Both these reasons practically exclude a possibility to measure sensor shifts out of production site, e.g. in a hospital.

In similar situations when dismantling is not desirable indirect methods are used. For example, it is known a method for scanner sensor geometrical shift measuring in an x-ray image of a test chart [patent US № 6600568, issued 29.07.2003]. This method involves scanning a test device having an image of a specific object, in the scan one selects some areas corresponding to different sensors; using their shifts sensor shifts are calculated.

In the claimed technical solution a method for measuring of geometrical sensor shifts in flat panel x-ray image sensors using a test device is considered. It is shown experimentally a possibility to specify geometrical sensor shifts using a test device having an x-ray transparent substrate and «sharp edge» (hereinafter, edge) test device. In the regions of interest (ROI) within the test device image the pixels which correspond to the edge of each test device are identified, data for calculations are generated, then geometrical sensor shifts are determined considering minimum value of an objective function.

A similar to the claimed method for measuring of sensor shifts is not known to the author from the state of the art.

**Summary of the invention**

A technical solution that the claimed invention is intended to solve consists in expansion of technical means for determining a sensor geometrical shift, more specifically, in developing a new method for determining sensor geometrical shifts using a test device that allows measuring with sufficient accuracy sensor shifts in flat panel x-ray detectors.

Technical result involves expansion of technical means for determining a sensor geometrical shift in flat panel x-ray detectors and a possibility to measure sensor geometrical shift with sufficient accuracy.

The said technical result is achieved in the method for determining a sensor geometrical shift in a flat panel x-ray detector having at least two sensors fixed on a mounting panel provided at the sensor butt there be a gap in between sensors, wherein the method consists in placing on the detector surface at least two edge test devices corresponding to a gap in between the said sensors; the said test device is exposed to x-rays in order to get its x-ray image; in the obtained image the pixels which correspond to the edge of each test device are identified, wherein these pixels are used to determine sensor geometrical shifts considering minimum value of an objective function.

To identify pixels corresponding to the edge image the image gradient in magnitude is calculated; pixels with the image gradient in magnitude higher than a given threshold value are identified; weighting factor- and pixel coordinates data are generated, wherein pixel gradient in magnitude is used as weighting factors.

The least-squares method with constraints on geometrical shifts is used as an objective function.

The surface of the test device performed of x-ray transparent substrate is marked by the lines corresponding to sensor butt-joints. There is one or more joint depending on a sensor number. On each of the said segments at least two edge test devices are placed in such a way that edges of adjacent test devices being perpendicular to each other, wherein the angle between the edge of each test device and appropriate segment is preferably 45 degrees and, wherein edges divide the said segment into inherently equal parts.

It is reasonable to make the test device substrate of organic glass.

The whole set of mentioned features allows achieving technical results that consist in determining sensor geometrical shifts with required accuracy.

**Detailed description of the invention**

Implementation of the method for determination of geometrical sensor shifts in flat panel detector is explained by the following drawings.

Fig. 1 shows an arrangement to implement the said method:

1 - x-ray tube;
2 - x-ray flow;
3 - x-ray image detector;
4 - test device .

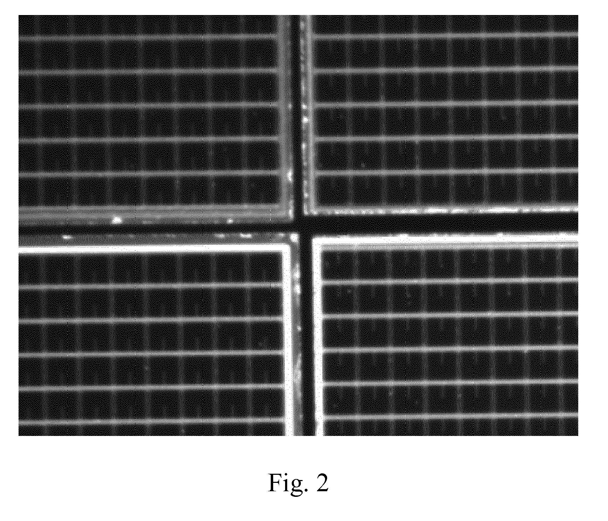

Fig.2 shows the sensors fixed on the common substrate. It is seen the sensors do not adjoin closely each other there is a gap between them.

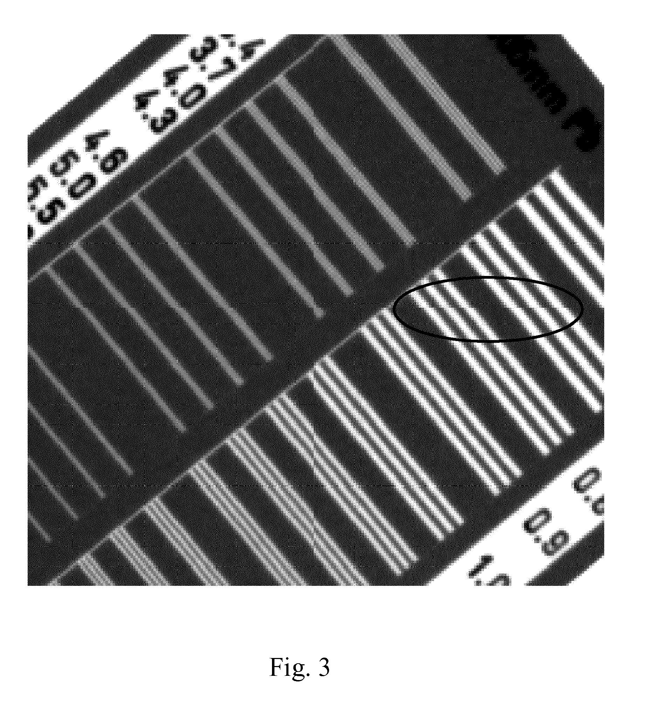

Fig. 3 shows a magnified part of the test device image (resolution target) at the sensors butt-joint. The oval marks the point where artifacts in the butt-joint area are most visible.

Fig. 4 shows schematic image of the test device 4 where:

*I , II* are image regions corresponding to sensors;
5 is a substrate;
6 is a line corresponding to sensors butt-joints;
7-8 are edge test devices and appropriate ROIs.
The test device is used to determine sensor shifts of a detector consisting of two sensors.

Fig. 5 shows schematic image of the test device 4 where:

*I - IV* are image regions corresponding to sensors;
5 is a substrate;
6, 15 is a line corresponding to sensors butt-joints;
7-14 are edge test devices and appropriate ROIs. The test device is used to determine a sensor shift of a detector consisting of four (2x2) sensors.

Fig. 6 shows the edge test device image gradient in magnitude.

Fig. 7 shows a part of the test device image with butt-joints of the neighbouring sensors. The points mark the pixels which are used to calculate a sensor shift. Pixels are numbered along horizontal and vertical axes.

Fig. 8 shows explanations for identification of the line on the base of a set of points. The points represent a set of data ($x$, $y$) used to build the said line with parameters ($p$, $\theta$).

Fig. 9 shows a detector MTF (modulation transfer function), horizontal axis means spatial frequency in mm $^{-1}$, vertical axis means MTF values.

Fig. 10 shows a histogram of $x$ - axis shift absolute error values. Horizontal axis means absolute error in pixels, vertical axis means corresponding probability values in percents.

Fig. 11 shows a histogram of $y$ - axis shift absolute error values, vertical axis means corresponding probability values in percents.

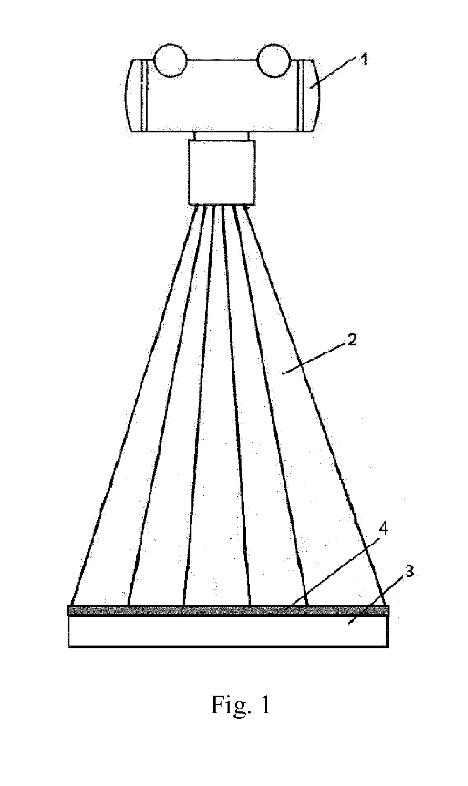

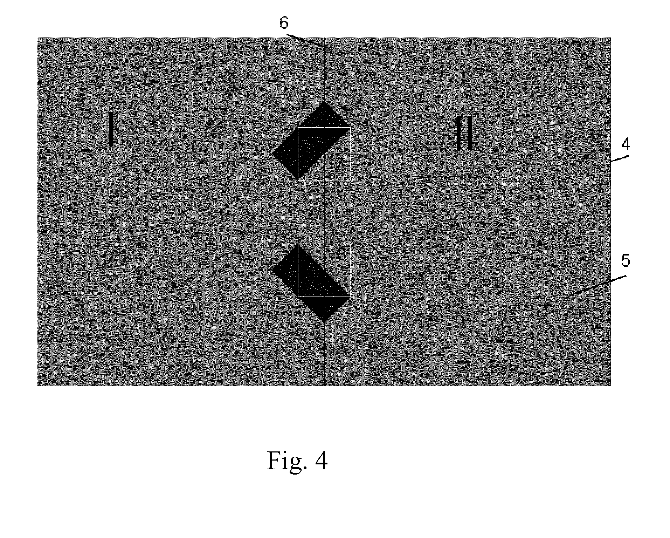

An x-ray image is obtained by an arrangement shown in Fig. 1. The arrangement comprises an x-ray tube 1. X-rays 2 are directed to the field of view of detector 3 where is placed a test device 4. Detector 3 comprises a scintillation screen (not shown), optically coupled with the detector active surface. The scintillation screen converts x-rays 2 into visible light, detector sensors convert them into digital image. According to claimed method on the field of view of detector 3 comprising at least two sensors fixed on a mounting plate provided at the sensor butt there be a gap in between sensors the test device 4 (Fig. 4) is placed. X-rays 2 are directed to the field of view of detector 3 and acquisition of an x-ray image of the test device occurs.

Let us describe the method of determination of geometrical sensor shifts in a flat panel detector using an x-ray image of the test device.

The image of the edge being approximated by a line shall have sufficient accuracy. The essence of the method consists in the following stages:

1) For each ROI is generated a set of data consisting of pixel coordinates and weighting factors corresponding to an edge image. Modulus of the gradient of an appropriate pixel is used as a weighting factor.

2) The sum of weighted squared residual is used as an error or as an objective function.

Let us describe a method for data generation for each ROI (Fig. 4, pos. 7-8). To calculate an image gradient in magnitude [Gonzalez et al., Digital image processing using MATLAB, p. 384, Prentice Hall, 2004] we use one-dimensional filter with radius $r$,

$$f = x \times \exp\left(-\frac{x^2}{2\sigma^2}\right) \quad x = [-r,...,r]$$

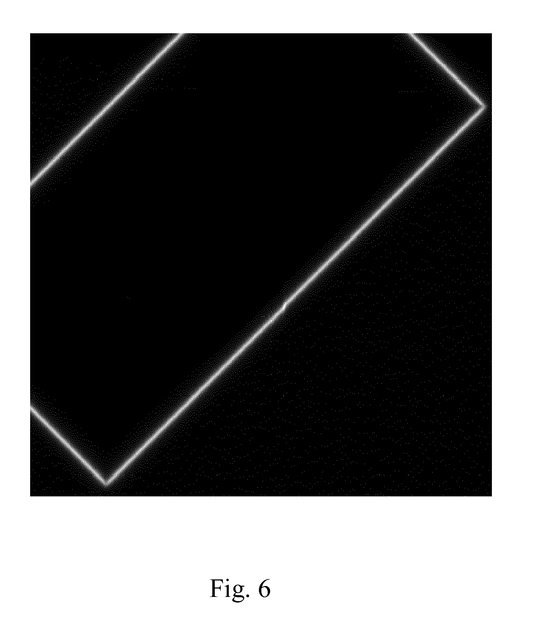

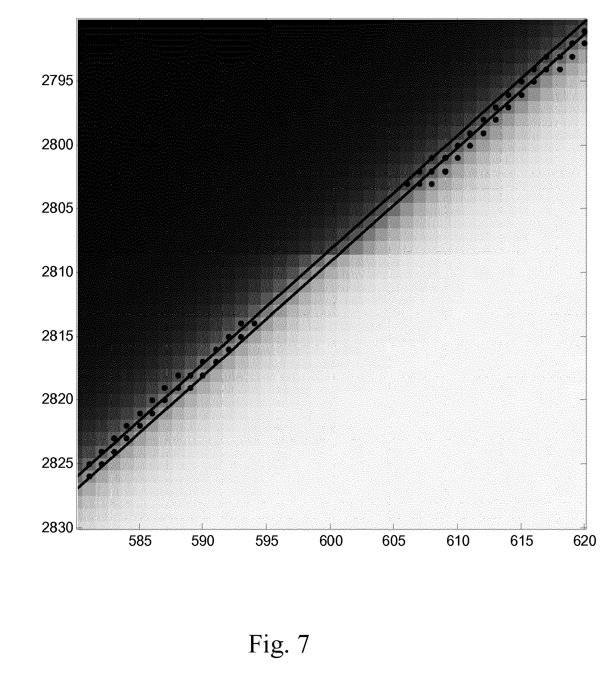

Each pixel $(x_i, y_i)$ has weight $\omega_i$, equal to modulus of the gradient. Let us further use only those pixels the weights of which are higher than the given threshold value $k \times \omega_{max}$ concerning maximum pixel value $\omega_{max}$ in the appropriate ROI. The constant $k$ and line filter parameters $(r,\sigma)$ are chosen in cause of numerical experiments. Fig. 6 shows an image part of the gradient in magnitude. Fig. 7 shows that the points mark the pixels the weights of which are higher than the given threshold value.

Let us describe the method to identify of line using data $(x_i, y_i, \omega_i)$, where $(x_i, y_i)$ are coordinates, $\omega_i$ are pixel weights. The parametric equation of a segment $(p, \theta)$ will be the following:

$$p + x \times \cos\theta + y \times \sin\theta = 0$$

The line parameters $(\theta, p)$ are determined from minimum of the function

$$E(\theta, p) = \sum_i \omega_i \times (p + x_i \times \cos\theta + y_i \times \sin\theta)^2$$

That is the sum of weight average squares of the distance from each pixel to the line $(\theta, p)$. The same function can be written in a matrix form

$$E(\theta, p) = \sum_i \omega_i \times (p + \tau \times X_i)^2$$

where $\tau = (\cos\theta, \sin\theta)$ and $X_i = (x_i, y_i)^T$. Parameter values $\theta$ and $p$, bringing the minimum to the function $E(\theta, p)$ are calculated in the following manner

$$\left\{ \begin{array}{l} \tan 2\theta = -\dfrac{2\sum\limits_{i} \omega_i \times (x_i - \bar{x}) \times (y_i - \bar{y})}{\sum \omega_i \times \left( (y_i - \bar{y})^2 - (x_i - \bar{x})^2 \right)} \\[4mm] p = -\dfrac{\cos\theta \times \left(\sum \omega_i \times x_i\right) + \sin\theta \times \left(\sum \omega_i \times y_i\right)}{\sum \omega_i} \end{array} \right.$$

where $\quad \bar{x} = \dfrac{\sum \omega_i \times x_i}{\sum \omega_i}$ , $\bar{y} = \dfrac{\sum \omega_i \times y_i}{\sum \omega_i}$ , they are determined from the condition that first derivatives of $E$

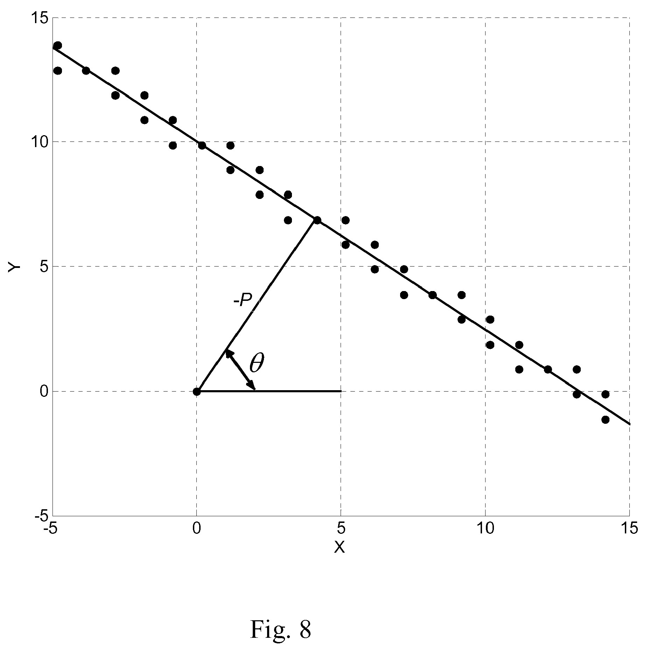

$(\theta, p)$ are equal to zero. Fig. 8 gives explanations to identification of a line using a given points set.

Let us describe the next stage of determination of the sensors shifts. Let $\left( x_i^{R,S}, y_i^{R,S} \right)$ and $\omega_i^{R,S}$ be pixel coordinates and weights belonging to ROI ($R$) and sensor ($S$). Let us introduce a global coordinate system connected with the left upper corner of sensor $I$ (sensor one) within which we shall perform all our calculations. Consider transformation of the Cartesian coordinates in the form

$$\widetilde{X} = O \times X + D$$

Here, matrix $O$ and vector $D$ determine a linear transformation of the coordinates

$$O = \begin{vmatrix} \cos\varphi & -\sin\varphi \\ \sin\varphi & \cos\varphi \end{vmatrix} \qquad D = \begin{pmatrix} d_x \\ d_y \end{pmatrix}$$

Consider the objective function

$$E_{I,II} = \sum_R \left( \sum_i \omega_i^{R,I} \left( p_R + \tau_R \times X_i^{R,I} \right)^2 + \sum_i \omega_i^{R,II} \left( p_R + \tau_R \times \left( O_{II} \times X_i^{R,II} + D_{II} \right) \right)^2 \right)$$

here $\tau_R = (\cos\theta_R, \sin\theta_R)$ and $\quad X_i^{R,S} = \left( x_i^{R,S}, y_i^{R,S} \right)^T$ , $S = I, II$ is index of sensors,

$R$ = 7, 8 is index of ROIs. Parameters $(p_7, \theta_7)$ и $(p_8, \theta_8)$ corresponding to the  object edges 7 and 8, rotation matrix $O_{II}$ and vector $D_{II}$ of the second sensor via the first one are determined by the minimizing of the objective function $E_{I,II}$ .To exclude solutions with sensor overlapping values $O_{II}$ and $D_{II}$ shall have additional constraints. Since the angles of sensor rotation are relatively small, assume they are equal to zero and constraints turn out to be especially simple:

$$E_{I,II} \rightarrow \min$$

$$D_{II,x} \geq 0$$

**The best embodiment of the invention**

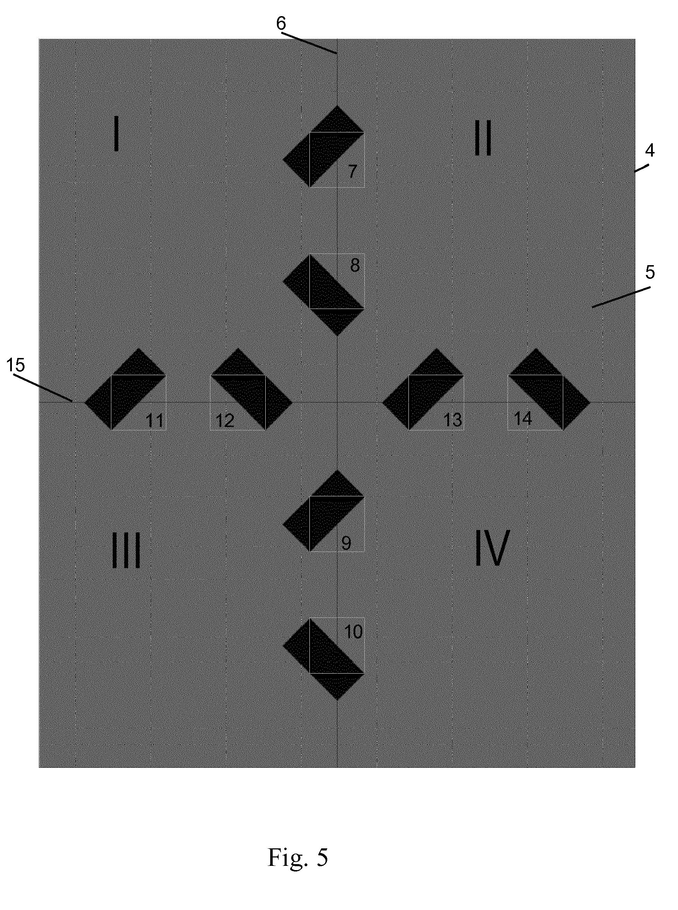

To determine sensor shifts in a flat panel detector comprising four (2x2) sensors test device 4 (Fig. 5) is placed in the field of view of the detector 3. Fig5 shows schematic image of the test device where: *I - IV* are image regions corresponding to sensors, pos. 7-14 are "edges". ROIs used to calculate sensor shifts are marked by frames. Test device 5 is a substrate performed of x-ray transparent substrate e.g., organic glass, of size corresponding to a particular detector size 3.. The lines 6 and 15 corresponding to the joints of the sensors are marked on the substrate 5.. On the said lines the "edges" 7-14 are fixed according to the technological gaps positions. The "edge" is a tungsten plate having a linear sharp edge; its dimensions are 20x10x1mm wherein pixel size is $50\mu$m. Such a plate is, for example, used for MTF estimation method in x-ray detectors [IEC 62220-1, First edition 2003-10]. Tungsten plates are fixed on the lines 6 and 15 of the substrate 5. The best way of plate position is when the edges of adjacent plates being perpendicular to each other, wherein the angle between the edge of plate and appropriate segment is preferably 45 degrees and, wherein edges divide the said segment into inherently equal parts.

To determine the geometry of the whole detector we shall minimize the objective function

$$E = E_{I,II} + E_{I,III} + E_{III,IV} + E_{II,IV}$$

$E_{I,II}$ determines the second sensor position via the first one (ROIs 7 and 8), $E_{I,III}$ determines the third sensor position via the first one (ROIs 11 and 12), $E_{III,IV}$ determines the fourth sensor position via the third one (ROIs 9 and 10), $E_{II,IV}$ determines the fourth sensor position via the second one (ROIs 13 and 14). Fig. 5 shows numbering of ROIs 7-14 and numbering of sensors *I - IV*. As a result we get the following problem with constraints

$$E \to \min$$

$$\begin{cases} D_{II,x} \geq 0 \\ D_{III,y} \geq 0 \\ D_{IV,y} \geq D_{II,y} \\ D_{IV,x} \geq D_{III,x} \\ D_{IV,x} \geq 0 \quad \text{or} \quad D_{IV,y} \geq 0 \\ D_{II,x} \geq D_{III,x} \quad \text{or} \quad D_{III,y} \geq D_{II,y} \end{cases}$$

(1) is a constraint for the second sensor shift via the first one, (2) is a constraint for the third sensor shift via the first one, (3) for the fourth sensor shift via the second one (4) for the fourth sensor shift via the third one. (5) is a constraint for the fourth sensor shift via the first one; (6) for the third sensor shift via the second one. To solve such a problem the standard gradient methods for numerical minimization of nonlinear tasks with constraints are used.

As mentioned above, a flat panel detector is an all-of-a-piece device that does not allow direct measuring shifts in between sensors. So, the functionality of the claimed method was tested using simulated images. There were 16 byte test device images simulated with a known sensor shift, the image characters were as follows:

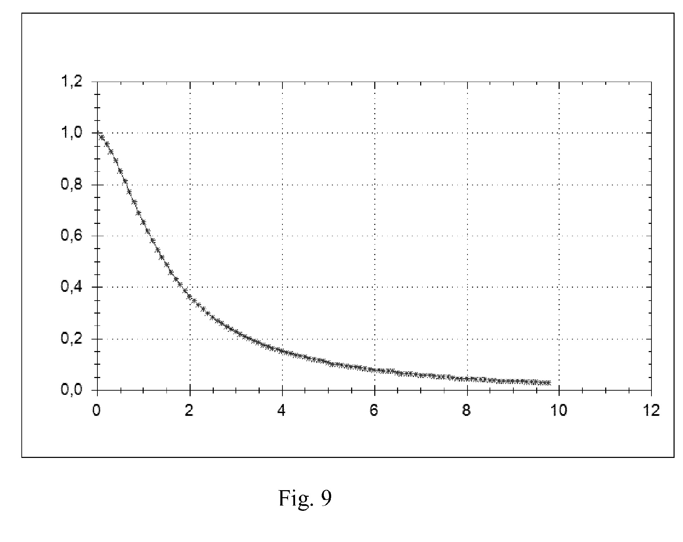

    1) Signal/noise levels in air image 30000 and 50 units, respectively.
    2) Signal/noise levels in tungsten plate image 650 and 15 units, respectively.
    3) The MTF of simulated images corresponds with that measured on real image; shown in Fig. 9. MTF measuring technique corresponds with. IEC 62220-1, First edition 2003-10.

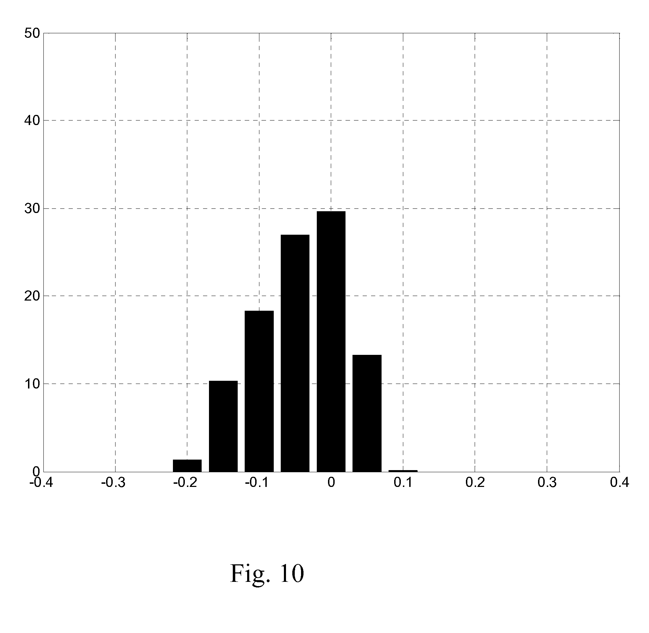

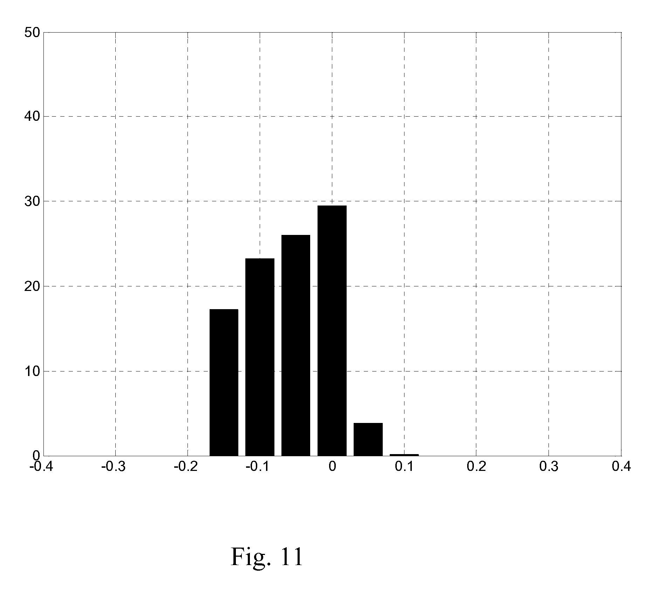

The simulated image is subject to superposition of noise corresponding to white noise having a normal distribution. Indicated values correspond to a real x-ray image of the test device. Sensor shifts $(\tilde{D}_{S,x}, \tilde{D}_{S,y})$ were generated by a random-number generator having a uniform distribution within $\pm 2$ pixel range. After that by means of the claimed method using simulated images there were calculated sensor shifts $(D_{S,x}, D_{S,y})$ which were compared with the original shift values $(\tilde{D}_{S,x}, \tilde{D}_{S,y})$. Numerical experiments show that the claimed method provides determination of sensor shifts in a flat panel detector with an absolute error within $\pm 2$ pixel range. Fig. 10 and 11 represent absolute error histogram $\varepsilon_x = \tilde{D}_{S,x} - D_{S,x}$ along $x$ -axis and $\varepsilon_y = \tilde{D}_{S,y} - D_{S,y}$ along $y$ .-axis

Utilization of the claimed method for determination of geometrical sensor shifts in an x-ray flat panel detector using a test device provides simple, effective, high accurate estimation of geometrical sensor shift avoiding detector dismantling.

The suggested method involves expansion of technical means of particular application.

**Industrial applicability**

The above description of the invention characterized in the independent claim involves a possibility of its realization by the use of mentioned in the said description and well-known tools and techniques. Therefore, the claimed method matches industrial applicability criterion.

The suggested technical solution is disclosed in the description accompanying with possible examples of its accomplishing which shall be considered method illustrations but not its limitation. On the base of given description qualified specialists have a possibility to suggest other versions within the patent claim.

**Claims**

1. Method for determination of geometrical sensors shift in flat panel x-ray image detectors, having at least two sensors fixed on a mounting panel , wherein the method consists in placing on the detector's operating surface at least two edge test devices corresponding to a gap in between the said sensors; the said test device is exposed to x-rays in order to get its x-ray image; in the obtained image the pixels which correspond to the edge of each test device are identified, wherein these pixels are used to determine sensor geometrical shifts considering minimum value of an objective function

2. The method of claim 1, wherein to identify pixels corresponding to the edge image the image gradient in magnitude is calculated; pixels with the image gradient in magnitude higher than a given threshold value are identified; weighting factor- and pixel coordinates data are generated, wherein pixel gradient in magnitude is used as weighting factors..

3. The method of claim 1, wherein the least-squares method having extra limits on geometrical shifts is used as an objective function.

4. The method of claim 1, wherein the surface of the test device performed of an x-ray transparent substrate is covered by markings in the form of lines corresponding to sensor butt- joints wherein there is one or more joints depending on a sensor number; on each of the said segments at least two edge test devices are placed in such a way that edges of adjacent test devices being perpendicular to each other, wherein the angle between the edge of each test device and appropriate segment is preferably 45 degrees and, wherein edges divide the said segment into inherently equal parts.

Fig. 1

Fig. 2

Fig. 3

Fig. 4

Fig. 5

Fig. 6

Fig. 7

Fig. 8

Fig. 9

Fig. 10

Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6895077 B
- US 7663115 B
- US 8073191 B
- US 6600568 B